# EUROPEAN PATENT APPLICATION

(11) **EP 3 407 569 A1**
(43) Date of publication of application: **28.11.2018**
(21) Application number: 18174044.0
(22) Date of filing: 24.05.2018
(51) Int. Cl.: H04L 29/08, H04W 84/02

(54) **DISTRIBUTED COMMUNICATION SYSTEM AND METHOD FOR CONTROLLING SUCH SYSTEM**

(30) Priority: 24.05.2017 NL 2018977
(71) Applicant: CLB Research B.V., 1483 GE De Rijp (NL)
(72) Inventor: ROOS, Raoul Jan Paul, 1483 GE De Rijp (NL)
(74) Representative: van Trier, Norbertus Henricus Gerardus

(57) **Abstract**

The invention relates to a distributed communication system comprising: one or more sources configured to communicate one or more communications when triggered thereto; one or more communicators configured to receive communications from the sources, and to signal the received communications to one or more recipients; and a control device configured to track communications originating from the sources, wherein the sources are configured to include an identificator into each communication originating therefrom, and to transmit for each communication a message with the associated identificator to the control device, wherein the communicators are configured to transmit an acknowledgment to the control device regarding signaling of a received communication, including the identificator associated with the signaled communication, and wherein the control device is configured to track communications within the distributed communication system by means of received associated identificators and to raise an alarm condition upon non-arrival of associated communications at any of the communicators.

## Description

### BACKGROUND

The invention relates to a distributed communication system for establishing communication in a care environment, wherein the distributed communication system comprises one or more sources configured to communicate one or more communications when triggered thereto; and one or more communicators configured to receive one or more communications from the one or more sources, and to signal the received one or more communications to one or more receivers.

Such a distributed communication system is for example used in hospitals. The distributed communication system comprises a number of sources, also known as interface units, which are provided in different patient rooms and are thus spread throughout the entire hospital. The interface units are connected to patient monitoring devices, such that when a patient monitoring device detects a life-threatening situation, it can trigger the connected interface unit to communicate an alarm communication to a care giver. The alarm communication is communicated via a number of network components, which form the network within the hospital, to a communicator such as a mobile phone or a pager of a care giver or an operator. The care giver or operator becomes aware of the life-threatening situation via the communicator and can respond to the situation.

A disadvantage of such distributed communication system is that one or more of the data communications within the distributed communication system can become disrupted, one or more communications can be lost within the distributed communication system or one or more of the interface units or network component can fail, such that the receivers do not receive a communication. In a care environment, failure of delivering such communication can lead to life-threatening situations.

It is an object of the present invention to ameliorate or to eliminate one or more disadvantages of the prior art, or to at least provide an alternative distributed communication system.

### SUMMARY OF THE INVENTION

According to a first aspect, the invention provides a distributed communication system for establishing communication in a care environment, wherein the distributed communication system comprises:
- one or more sources configured to communicate one or more communications when triggered thereto;
- one or more communicators configured to receive one or more communications from the one or more sources, and to signal the received one or more communications to one or more recipients; and
- a control device configured to track communications originating from the one or more sources,
wherein the one or more sources are configured to include an identificator into each communication originating therefrom, and to transmit for each communication a message with the associated identificator to the control device,
wherein the one or more communicators are configured to transmit an acknowledgment to the control device regarding signaling of a received communication, including the identificator associated with the signaled communication,
and wherein the control device is configured to track communications within the distributed communication system by means of received associated identificators and to raise an alarm condition upon non-arrival of associated communications at any of the one or more communicators.

The distributed communication system according to the invention has a control device which is adapted to raise an alarm condition when a communication which is communicated by one of the one or more sources for example gets lost on the way to at least one of the one or more communicators. Due to the raised alarm condition, it is indicated to for example an operator of the distributed communication system that something is wrong within the distributed communication system and that communications possibly do not reach the intended care giver(s) or operator(s). In response to the raised alarm condition, appropriate measures can be taken such that life-threatening situations due to a communication failure between the one or more source and the one or more communicators are prevented. So, due to the distributed communication system according to the invention, it is clear to operators and/or care givers all the time whether or not the distributed communication system is functioning properly, therewith reducing the risk of, or in the ideal case preventing life-threatening situations due to a communication failure.

In an embodiment the control device comprises a receiver configured to receive each message from the one or more sources, and each acknowledgement from the one or more communicators. In a further embodiment the control device is configured to maintain a list of active communications, wherein a communication is entered onto the list upon receipt of the corresponding message with identificator and, optionally, is removed from the list upon receipt of the corresponding acknowledgement. An advantage of this embodiment is that the control device has an overview of active communications. Preferably, the list of active communications can be outputted to for example a display of an operator, such that the operator is able to see what kind of communications are communicated at any specific moment but still have to be delivered to a care giver.

In an embodiment the receiver further is configured to check the integrity of each message from the one or more sources and of each acknowledgement of the one or more communicators, and preferably to raise an alarm condition when a checked message and/or acknowledgement is invalid. If a message is corrupted or is incomplete, the integrity thereof is incorrect and the message is therefore considered to be invalid. If a message is invalid, this can be caused by a communication failure due to which the communication communicated within the distributed communication system became corrupted or incomplete. Such corrupted or incomplete message can provide false information, which can lead to the impression that the distributed communication system is working correctly, while this is not the case. This can lead to the situation that an alarm situation is occurring at the source side, while the care giver is of the impression that everything is fine. By checking the integrity of each message and each acknowledgement, the risk of this situation is reduced or in the ideal case eliminated, since a corrupted message causes the alarm condition to be raised.

In an embodiment each message from the one or more sources additionally comprises at least one of a sender ID and a timestamp. In a further embodiment the control device is configured to verify timely signaling of each communication from the one or more communicators, preferably by means of determining an expired amount of time between receipt of each of the identificators and receipt of the corresponding acknowledgement, and to raise an alarm condition when one or more communications from the one or more communicators are not timely signaled. When the amount of time between communicating a communication by one of the sources and signaling of the communication by one of the communicators exceeds a given threshold, such as five seconds, this can lead to life-threatening situations. For example in the case of a heart failure, it is important that care givers respond as fast as possible and a delay of the communication indicating the heart failure can be detrimental or even deadly to the patient. When care givers are made aware of such delay by means of the raised alarm condition, precautionary measures can be taken in order to prevent detrimental or life-threatening situations.

In an embodiment the control device is configured to verify correct signaling of each communication from the one or more communicators and/or correct communicating of each communication from the one or more sources, and to raise an alarm condition when one or more communications from the one or more communicators are not correctly signaled, and/or when one or more communications from the one or more sources are not correctly communicated. If a communication is incorrectly communicated or signaled, this can be caused by a communication failure due to which the communication became corrupted or incomplete. Such corrupted or incomplete communication can provide false information, which can lead to the impression that the distributed communication system is working correctly, while this is not the case. This can lead to the situation that an alarm situation is occurring at the source side, while the care giver is of the impression that everything is fine. By the verification by the control device, the risk that such situation occurs is reduced or in the ideal case eliminated.

In an embodiment the control device comprises a verifier configured to verify timely and/or correct signaling of each communication from the one or more communicators and/or correct communicating of each communication from the one or more sources. By separating the functionalities of receipt of the messages by the control device and of verification of the communications by the control device, the risk of a delay in processing the messages from one or more of the sources and the acknowledgements from the one or more communicators is reduced.

In an embodiment the control device comprises a logger and a storage for logging and storing messages received from the one or more sources and acknowledgements received from the one or more communicators. By logging and storing all messages and acknowledgements received by the control device, it is possible to perform an analysis onto the stored messages and acknowledgements. An operator of the distributed communication system can for example determine how long it takes on average to signal a communication to a recipient starting from the moment of communicating the communication by a source. This embodiment of the invention enables the operator to gain insight about the functioning of the distributed communication system.

In an embodiment the distributed communication system further comprises an indicating device for indicating when an alarm condition has been raised. The indicating device can be in the form of a signalling lamp with two clusters of LED's, respectively coloured red and green. When the red coloured cluster of LED's is activated, care givers or operators can see that something is wrong within the distributed communication system. When the green coloured cluster of LED's is activated, it is an indication that the distributed communication system is working properly. An advantage of this embodiment is that it is easy to see whether or not the distributed communication system is working properly.

In an embodiment the distributed communication system further comprises one or more integrators, wherein each of the one or more integrators is in data communication with the one or more sources, with the one or more communicators, and/or with one or more other integrators, wherein each of the one or more integrators is configured to receive at least one communication originated by at least one of the one or more sources and/or by at least one of one or more other integrators, and to forward the at least one received communication to the one or more communicators, and/or to one or more other integrators. In an embodiment thereof each of the one or more integrators is configured to send a message to the control device to confirm that the at least one received communication has been forwarded or has been received, wherein the message comprises the identificator of the respective communication. An advantage of this embodiment is that the path of each communication can be followed in detail. Thus when the distributed communication system is not working properly, it can be seen which integrators (network components) are passed by the communications and which are not, so the operator can get an indication of the position within the distributed communication system where the communication(s) get lost.

In an embodiment the distributed communication system comprises one or more sensors configured to verify the functioning of the one or more communicators, preferably wherein the one or more sensors are configured to raise an alarm condition when a communication failure occurs by at least one of the one or more communicators. An advantage of this embodiment is that an end-to-end check of the distributed communication system is provided.

In an embodiment at least one of the one or more communicators is configured to support operator confirmation, preferably wherein the at least one of the one or more communicators is configured to transmit the operator confirmation to the control device. An advantage of this embodiment is that the distributed communication system is provided with a confirmation that a care giver has received and seen a communication, therewith providing a complete check of the distributed communication system.

In an embodiment each of the one or more sources comprises a source ID, wherein each identificator included by the one or more sources comprises an associated source ID and a local ID, preferably wherein a value of the local ID is incremented or decremented each time a subsequent identificator is included in a message, preferably wherein the value of the local ID is sequential. A number of communications communicated by a source can for example have the following identificators: B12-001, B12-002, B12-003, ..., B12-N, wherein N is an integer, B12 is the source ID and 001, 002, 003, ..., N are the local ID's. By using a sequential incrementing or decrementing value for the local ID, it is immediately visible when a communication has been missed within the distributed communication system. When for example the following identificators are received by the control device: B12-001, B12-003, ..., B12-N, it is clear that the communication with B12-002 as identificator is missed. Missing a communication can lead to life-threatening situations as explained before. An advantage of including identificators as described above, is that it is relatively simple to check whether all communications are correctly communicated within the distributed communication system.

In an embodiment the one or more sources are configured for communicating a test communication, preferably periodically, and the one or more communicators are configured to acknowledge receipt of such test communication, preferably automatically. By sending a test communication to the one or more communicators which acknowledge receipt thereof automatically to the control device, the control device is able to keep an overview of communicators which are reachable or not. In case one of the communicators is not reachable, for example due to telephone network issues, the control device can raise an alarm condition. It is therewith prevented that the operator has the impression that recipients are receiving the signaled communications, while this is not the case.

In an embodiment the distributed communication system is a distributed alarm system.

According to a second aspect, the invention provides a method of controlling a distributed communication system, wherein the distributed communication system comprises:
- one or more sources configured to communicate one or more communications when triggered thereto;
- one or more communicators configured to receive one or more communications from the one or more sources, and to signal the received one or more communications to one or more recipients; and
- a control device configured to track communications originating from the one or more sources, wherein the method comprises the steps of:
- at each source including an identificator into each communication originating therefrom;
- for each communication, transmitting a message with the associated identificator to the control device;
- transmitting an acknowledgment to the control device regarding signaling of a received communication, including the identificator associated with the signaled communication;
- tracking communications within the distributed communication system by means of received associated identificators; and
- raising an alarm condition upon non-arrival of associated communications at any of the one or more communicators.

The method according to the invention provides at least the same advantages as mentioned in relation to the distributed communication system according to the first aspect of the invention.

In an embodiment each of the one or more sources comprises a source ID, wherein the step of at each source including an identificator into each communication originating therefrom comprises the step of combining the source ID and a local ID into an identificator.

In an embodiment the local ID has a value, wherein the method further comprises the step of incrementing or decrementing the value of the local ID for each subsequent identificator to be included, preferably wherein the local ID is sequential.

In an embodiment the control device is configured to maintain a list of active communications, wherein the method comprises the steps of adding a communication to the list of active communications upon receipt of a message from the one or more sources and closing the communication upon receipt of an acknowledgement of the one or more communicators acknowledging signaling of the associated communication.

In an embodiment the distributed communication system comprises one or more integrators, wherein each of the one of more integrators is in data communication with the one or more sources, with the one or more communicators, and/or with one or more other integrators, wherein each of the one or more integrators is configured to receive at least one communication originated by at least one of the one or more sources and/or by at least one of one or more other integrators, and to forward the at least one received communication to the one or more communicators, and/or to one or more other integrators, preferably wherein each of the one or more integrators is configured to send a message to the control device to confirm that the at least one received communication has been forwarded or has been received, wherein the message comprises the identificator of the respective communication.

In an embodiment the method comprises the step of updating the list of active communications upon receipt of a message of the one or more integrators, such that it is indicated in the list of active communications that the communication associated with the received message has reached or passed the integrator which has sent the message.

According to a third aspect, the invention provides a computer-readable medium having computer-executable instructions adapted to cause a distributed communication system according to the first aspect of the invention to perform a method according to the second aspect of the invention.

The various aspects and features described and shown in the specification can be applied, individually, wherever possible. These individual aspects, in particular the aspects and features described in the attached dependent claims, can be made subject of divisional patent applications.

### BRIEF DESCRIPTION OF THE DRAWINGS

The invention will be elucidated on the basis of an exemplary embodiment shown in the attached drawings, in which:
Figure 1 shows a schematic block schedule of an embodiment of a distributed communication system according to the invention;
Figure 2 shows a schematic block schedule of another embodiment of a distributed communication system according to the invention;
Figure 3 shows a detailed block schedule of a further embodiment of a distributed communication system having a control device according to the invention; and
Figure 4 shows a schematic overview of an embodiment of a distributed communication system implemented in a care environment.

### DETAILED DESCRIPTION OF THE INVENTION

A distributed communication system 1 according to the invention is intended to be used in a care environment, such as a hospital, a psychiatric institution or a prison. When used in a hospital or a psychiatric institution, such a distributed communication system is also known as a 'nurse call system' or a distributed alarm system. In such environments, human care givers are assisted by such a distributed alarm system, wherein the distributed alarm system allows the human care givers to take care of multiple persons whom are or may become in the need of specific care. For example, when a patient in a hospital is monitored by means of a heart rate monitor and the heart of the patient fails, the heart rate monitor is configured to signal an alarm signal towards a remote human care giver. This alarm signal is sent to the distributed alarm system by the heart rate monitor, and the distributed alarm system delivers the alarm signal to the human care giver.

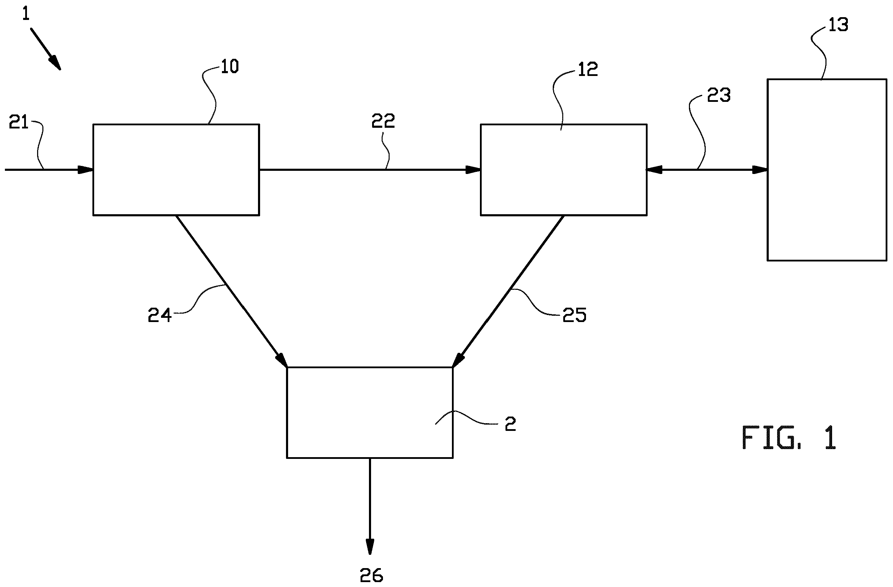

As schematically illustrated in figure 1, such a distributed alarm system 1 comprises a source 10 and a communicator 12, wherein the communicator 12 is in data communication with the source 10 via a first data connection 22. The first data connection 22 can be formed by a wireless data connection, a wired data connection or a combination thereof.

The source 10 is configured to receive a trigger communication 21 from a person pushing a non-shown button on the source or a trigger communication 21 from a non-shown device, such as a heart rate monitor, indicating an alarm condition, such as heart failure of a patient monitored by the heart rate monitor. Following receipt of the trigger communication 21, the source 10 issues a communication, such as an alarm signal, which is intended to make a care giver or another system aware of an alarm situation occurring at the source side. The communicator 12 is configured to receive the alarm signal issued by the source 10 via the first data connection 22. Subsequently, the communicator 12 signals the alarm signal towards a care giver and/or alarming system 13 via a communication line 23, here schematically shown as a block, via which the care giver becomes aware of the alarm situation at the source side or the alarming system starts broadcasting an alarm. The care giver or an operator can confirm that the alarm signal has been received and noticed.

Although only a single source 10 and a single communicator 12 are shown, it is noted that multiple sources 10 and multiple communicators 12 can be provided within such distributed alarm system 1. Each source 10 can be connected to one or more communicators 12, such that each alarm signal from one of the sources 10 can be send to multiple communicators 12. Each of the communicators 12 can signal an alarm signal to one or more care giver 13, such that one or multiple care givers become alerted. It can be dependent on the kind of alarm signal whether one or multiple care givers receive the alarm signal.

It might happen that the first data connection 22 between for example the source 10 and the communicator 12 becomes disrupted, or that one of the source 10 and the communicator 12 fails for whatever reason. Such disruption or failure can remain unnoticed for quite some time and can lead to life-threatening situations. In order to provide care givers or an operator an indication about whether the distributed communication system 1 is functioning properly, the distributed communication system 1 is provided with a control device 2 according to the invention.

As illustrated in figure 1 the control device 2 is in data communication with the source 10 and the communicator 12 via a third 24 and fourth 25 data connection, respectively. Furthermore, when the source 10 receives the trigger communication 21, the source 10 includes a unique identificator within the alarm signal issued following on receipt of the trigger communication 21. The unique identificator 24 is also transmitted towards the control device 2 via the third data connection 24. Upon receipt of the unique identificator, the control device 1 starts tracking the corresponding alarm signal. In order to track the corresponding alarm signal, at least a time of receipt of the unique identificator is recorded and a timer is started.

After receipt of the alarm signal with the unique identificator at the communicator 12 and after signaling of the alarm signal via data connection 23 towards the care giver 13, the communicator 12 transmits an acknowledgement thereof towards the control device 2. The acknowledgement includes a confirmation of successful signaling of the alarm signal, and includes the unique identificator. Upon receipt of the acknowledgement, the control device 2 confirms internally that the alarm signal is signaled to the care giver and stops tracking of the alarm signal. If the care giver 13 receives the alarm signal within a predetermined time and thus the control device 2 receives the acknowledgement of the communicator 12 within the predetermined time, the control device 2 issues an indication 26 that the distributed alarm system 1 is working properly. If the control device 2 does not receive the acknowledgement within the predetermined time, the control device 2 raises an alarm condition and issues an indication 26 relating to the raised alarm condition that the distributed alarm system 1 is not working properly. Due to the indication 26, the care givers or operators can become aware whether or not communication between the source 10, the communicator 12 and the care giver 13 is functioning properly. When it is indicated that for example a communication failure occurred between the source 10 and the communicator 12, the care giver 13 is aware of failure of the distributed alarm system 1 and can respond to this situation. Life threatening situations due to non-receipt of alarm signals are therewith reduced and in the ideal case prevented.

It is noted that the indication 26 originating from the control device 2 can be used to control a non-shown light assembly which is configured to emit for example green light when the distributed alarm system 1 is working correctly and to emit for example red light when the distributed communication system 1 is not working correctly.

Although not shown in figure 1, the distributed alarm system 1 may comprise sensors which are provided to sense whether the communicator 12 is functioning correctly. When such sensor senses for example that a communication failure occurred at the communicator 12, the sensor can raise an alarm condition preferably via the control device 2.

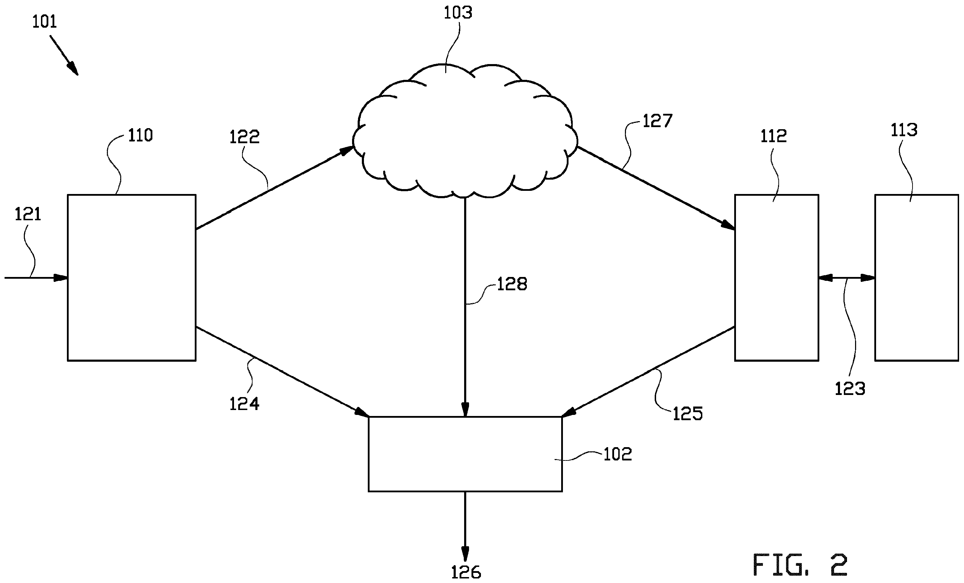

In figure 2 a block schedule of another embodiment of a distributed alarm system 101 according to the invention is illustrated. The distributed alarm system 101 comprises a source 110, a communicator 112 and a control device 102 which corresponds substantially to the source 10, the communicator 12 and the control device 2 as described in relation to the embodiment according to figure 1.

The distributed alarm system 101 further comprises one or more integrators which are illustrated as a network cloud 103. The network cloud 103 represents a network or a part thereof provided between the source 110 and the communicator 112 and can be formed by means of one or more non-shown network components, such as but not limited to switches, routers, servers, repeaters, gateways and/or phone masts.

In the embodiment shown in figure 2, the source 110 is configured to receive a trigger communication 121 as described in relation to the source 10 in figure 1. Following receipt of the trigger communication 121, the source 110 issues a communication such as an alarm signal via the first data connection 122 towards the network cloud 103, includes a unique identificator within the alarm signal and transmits the unique identificator to the control device 102 via the third data connection 124. Upon receipt of the unique identificator by the control device 102, the control device 102 starts tracking the corresponding alarm signal.

The one or more integrators within the network cloud 103 are configured to receive the alarm signal from the source 110. The integrators within the network cloud 103 receive - during use - a number of alarm signals originating from multiple sources 110. After receipt of each alarm signal, each of the integrators within the network cloud 103 processes the received alarm signal and forwards the alarm signal towards another integrator or via the second data connection 127 towards a communicator 112. The alarm signal comprises the unique identificator. Upon forwarding of a received alarm signal, each of the integrators within the network cloud 103 sends a message to the control device 102 via an fifth data connection 128. The message indicates to the control device 102 that the respective integrator is no longer handling the specific alarm signal.

Upon receipt of the alarm signal by the communicator 112, the alarm signal is forwarded to the care giver 113 via communication line 123. At the same time, the communicator 112 transmits an acknowledgement signal with the unique identificator to the control device 102 via the fourth data connection 125, which acknowledgement with identificator is processed by the control device as described in relation to the first embodiment according to figure 1.

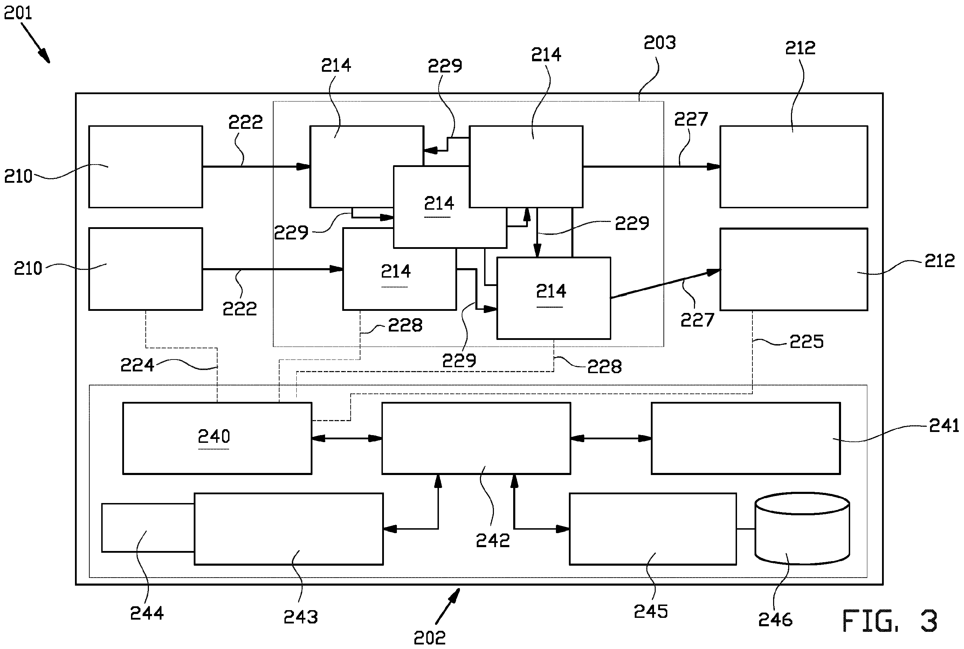

Figure 3 is illustrates a detailed block schedule of a further embodiment of a distributed alarm system 201 according to the invention. The distributed alarm system 201 comprises a number of sources 210, a network part 203 with a number of integrators 214, and a number of communicators 212, such as mobile phones and/or pagers. The sources 210 are in data communication with the integrators 214 via first data connections 222. The integrators 214 are in data communication with each other via intermediate data connections 229 and/or with the communicators 212 via second data connections 227. It is noted that the sources 210, the integrators 214 and the communicators 212 can be placed in different rooms and/or areas of the care environment. Moreover, each source 212 can be connected to the same or another integrator 214 via a separate first data connection 222, and each integrator 214 can be connected to the same or another communicator 212 via a separate second data connection 227. The sources 210, the integrators 214 and the communicators 214 function substantially the same as described in relation to the embodiment according to figures 1 and 2.

As best shown in figure 3 the control device 202 is provided with a receiver 240, a communicator 241, routing 242, a verifier 243 with plugins 244, a logger 245, and a storage 246. The receiver 240, the communicator 241, the verifier 243 with plugins 244, and the logger 245 are in data communication with each other via routing 242. The storage 246 is in data communication with the logger 245.

The communicator 241 is preferably connected with a non-shown signalling lamp with two clusters of LED's, respectively coloured red and green. Upon receipt of an alarm condition, the communicator 241 activates the red coloured cluster of LED's, such that care givers or operators can see that the distributed alarm system 201 is not functioning properly. When the green coloured cluster of LED's is activated, it is an indication that the distributed alarm system 201 is working properly. In the case that the control device 202 is not working properly, all LED's are de-activated.

The receiver 240 is adapted to be in data communication with each of the sources 210, the integrators 215 and the communicators 212 via third 224, fourth 225 and fifth 228 data connections, respectively. Each communication, signal or message of the components communicated to the control device 201 is received by the receiver 240, whereby each of the communications pertains to an alarm, which is uniquely identified by an identificator, and is provided with a timestamp and a sender ID. The sender ID corresponds to a unique identification within the distributed communication system 201 of the network component sending the corresponding communication. The unique identificator of the alarm is made up of the source ID and a local identificator, which is preferably strictly sequential. For example, when one of the sources 210 has as source ID A12 and includes a unique identificator into a communication for the first time, the unique identificator would be A12-001. The next time the unique identificator would be A12-002 and so on. Each communication is thus uniquely identified within the distributed communication system 201. The source ID and the sender ID are the same. Examples of sender ID's and unique identificators are shown in table 1 below.

It is noted that in an embodiment, the control device 202 is, optionally, configured to set the value of the local identificator of the sources 210, such that the local identificators issued by one of the sources 210 is within a specific range of numerical values, or to reset the value of the local identificator of the sources 210.

When the receiver 240 receives a message, the receiver 240 verifies the integrity of the respective message. If the integrity of the respective message is false, i.e. the message is corrupt, the receiver 240 raises an alarm condition and forwards this alarm condition towards the communicator 241 via the routing 242. Upon receipt of the alarm condition, the communicator 241 de-activates the green coloured cluster of LED's and activates the red coloured cluster of LED's.

If the message is correct, the receiver 240 subsequently checks the sender ID and the unique identificator which are incorporated in the message. When the unique identificator is unknown to the receiver 240 and the sender ID, which in this case corresponds to the source ID, is known to the receiver 240, the alarm is added to a list of active alarms as shown in table 1 below with a pending state. In the list 260, each active alarm is placed onto a row thereof and for each alarm the following properties are recorded: sender ID', 'ID (identificator)', 'time stamp', 'integrator (s) passed?', "time of acknowledgement' and 'status'. When the unique identificator is already known to the receiver 240, the status of the active alarm is updated. The update of the status of the active alarm can pertain to that an integrator 214 has forwarded the corresponding communication, for example by updating the number of integrators 214 which have forwarded the corresponding communication, or to an acknowledgment of that a communicator 212 has signalled the corresponding communication to a care giver. If it is acknowledged that the corresponding alarm signal of an active alarm is signalled to a care giver, so that the status of the active alarm becomes 'acknowledged', the alarm can be removed from the list of active alarms. As long as the state of the active alarm is 'pending', the corresponding communication is not signalled to a care giver yet. Table 1 shows an example of the list with active and acknowledged alarms, but this list can have any suitable shape.

**Table 1: List of active and acknowledged alarms.**

| **Sender ID** | **ID** | **Time stamp** | **Integrator(s) passed** | **Time of acknowledgement** | **Status** |
|---|---|---|---|---|---|
| A12 | A12-001 | 13.22.01 | 2 | 13.22.03 | Acknowledged |
| A12 | A12-002 | 15.12.13 | 3 | 15.12.15 | Acknowledged |
| A12 | A12-003 | 19.51.43 | 1 | 19.51.48 | Acknowledged |
| B12 | B12-001 | 20.01.33 | 4 | 20.01.34 | Acknowledged |
| \| | \| | \| | \| | \| | |
| C12 | C12-001 | 23.16.57 | 5 | 23.17.11 | Acknowledged |
| C12 | C12-002 | 03.12.23 | 0 | | Pending |
| | | | | | |

As mentioned above, the control device 202 comprises a verifier 243 with plugins 244 which is in data communication with the receiver 240 via the routing 242. The verifier 242 is configured to ensure that the communications which are represented at the list of active alarms are delivered timely. Timely delivery of each communication can be ensured by determining for example the time elapsed since the timestamp of the respective communication, or the time elapsed between the timestamp and receipt of the corresponding time of acknowledgement. When the determined time exceeds a predetermined threshold, the verifier 243 raises an alarm condition and transmits the alarm condition to the communicator 241. Upon receipt of the alarm condition, the communicator 241 de-activates the green coloured cluster of LED's and activates the red coloured cluster of LED's.

The verifier 243 is further configured to ensure the correct delivery of each communication, i.e. the communications are delivered completely and/or do not suffer from any other corruptions. The verifier 243 comprises the plugins 244 which cooperate with non-shown plugins at the sources 210, integrators 214 and the communicators 212 in order to guard the communications through the distributed alarm system 201.

The logger 245 and the storage 246 are provided for logging and storing all communications received and processed by the control device 202, so that for example a risk analysis can be performed on basis of the logged and stored communications, or to prove that the distributed alarm system 201 was working correctly at a given point of time.

It is noted that the distributed alarm system 201 is also capable of monitoring whether the intermediate data connections 229 between the integrators 214, or the integrators 314 themselves are functioning properly. In the case of failure of one or more of the intermediate data connections 229 and/or one or more of the integrators 214, the control device 202 will sense this and will raise an alarm condition.

Although it is not illustrated in figure 3, the control device 202 may request confirmation of a care giver that the communication has been received. Such confirmation can be given for example by pushing a button on the respective communicator 212, which communicator 212 transmits the confirmation to the control device 202. The confirmation includes the unique identificator and the sender ID of the respective communicator 212 and can be recorded in the list of active alarms and can be logged by the logger 245. Optionally, such confirmation also comprises a confirmation timestamp such that the control device 202 can determine how much time has elapsed since the timestamp of the respective source 210 and the confirmation timestamp. Optionally, when the control device 202 determines that the amount of time elapsed between the source timestamp and the confirmation timestamp exceeds a given threshold, the control device 202 can raise an alarm condition.

Optionally, a test communication can be send to the communicators 212 in order to see whether each of the communicators 212 is reachable. The test communication includes additional information to indicate to the communicator 212 receiving the test communication that it is unnecessary to signal the test communication to the care giver carrying the respective communicator 212, but to only acknowledge receipt of the test communication to the control device 202. The control device 202 therefor can maintain an overview about which communicators 212 are reachable.

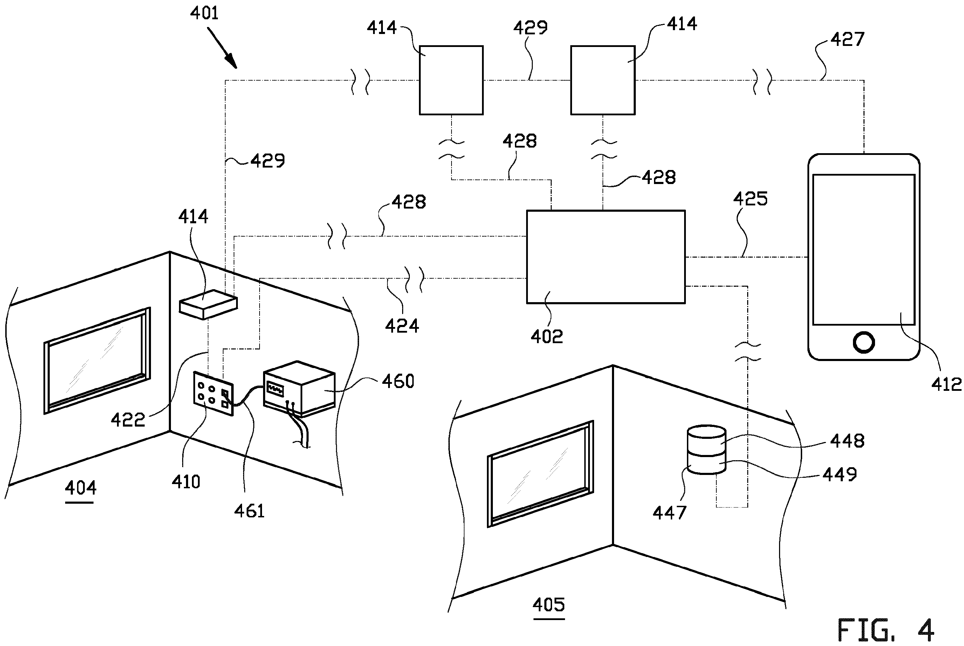

A schematic overview of an embodiment of a distributed alarm system 401 implemented in a hospital environment is shown in figure 4. The distributed alarm system 401 comprises sources 410, integrators 414, communicators 412 and a control device 402. The source 410 is in data communication with an integrator 414 via a first data connection 422 and with the control device 402 via a third data connection 424. The integrators 414 are in data communication with each other via intermediate data connections 429 and with the control device 402 via fifth data connections 428. The integrators 414 are in data connection with the communicators 412 via a second data connection 427. The communicators 412 are in data connection with the control device via a fourth data connection 425. Since the hospital has a number of patient rooms 404 and each of the patient rooms 404 comprises one or more sources 410, the integrators 414 are used to form a network with all available sources 410. The functioning of the integrators 414 is described in relation to the embodiments according to figures 2-3.

In this embodiment, the source of the distributed alarm system 401 is an interface unit 410 which is placed in a patient room 404 within a hospital. The interface unit 410 is connected to a heart rate monitor 460 via a data connection 461. When the heart rate monitor 460 detects a heart failure of a non-shown patient to which the heart rate monitor 460 is connected, the heart rate monitor 460 sends an alarm communication to the interface unit 410. The interface unit 410 includes a unique identificator into the alarm communication and forwards the alarm communication with the unique identificator to the integrator 414, and also transmits the unique identificator with sender ID and timestamp to the control device 402. The sender ID corresponds to the interface unit ID, wherein ID stands for identificator.

The communicator of the shown distributed alarm system 401 comprises a mobile phone 412. The mobile phone 412 is in data communication with one or more integrators 414 via the second data connection 427 and with the control device 402 via the fourth data connection 425. The functioning of the mobile phone 412 is described in relation to figures 1-3.

As illustrated in figure 4 the control device 402 is in data communication with a signalling lamp 447 with a red coloured cluster 448 of LED's and a green coloured cluster 449 of LED's. The signalling lamp 447 is placed in an operator's room or a security room 405, where people are located which are responsible for the security and other daily business of the hospital. As described in relation to figure 3, upon receipt of an alarm condition, the red coloured cluster 448 of LED's is activated and the green coloured cluster 449 of LED's is de-activated, such that care givers or operators can see that the distributed alarm system 401 is not working properly. When the green coloured cluster 449 of LED's is activated and the red coloured cluster 448 of LED's is de-activated, it is an indication that the distributed alarm system 401 is working properly. In the case that the control device 402 is not working properly, all LED's are de-activated. When the operators notices that all LED's are de-activated or that solely the red coloured cluster 448 is activated, the operators knows that the distributed alarm system in not working properly and can respond to it.

Additionally, an indication can be send to a number of or all communicators 412 within the distributed alarm system 401 in order to make the users thereof aware of the raised alarm condition.

It is noted that the components of the control device 402 can be implemented by using hardware components such as FPGA's or ASIC's, or by implementing the logic of at least some of the components into software which can be executed by a general computer or server.

It is to be understood that the above description is included to illustrate the operation of the preferred embodiments and is not meant to limit the scope of the invention. From the above discussion, many variations will be apparent to one skilled in the art that would yet be encompassed by the scope of the present invention.

## Claims

1. Distributed communication system for establishing communication in a care environment, wherein the distributed communication system comprises:
- one or more sources configured to communicate one or more communications when triggered thereto;
- one or more communicators configured to receive one or more communications from the one or more sources, and to signal the received one or more communications to one or more recipients; and
- a control device configured to track communications originating from the one or more sources,
wherein the one or more sources are configured to include an identificator into each communication originating therefrom, and to transmit for each communication a message with the associated identificator to the control device,
wherein the one or more communicators are configured to transmit an acknowledgment to the control device regarding signaling of a received communication, including the identificator associated with the signaled communication,
and wherein the control device is configured to track communications within the distributed communication system by means of received associated identificators and to raise an alarm condition upon non-arrival of associated communications at any of the one or more communicators.

2. Distributed communication system according to claim 1, wherein the control device comprises a receiver configured to receive each message from the one or more sources, and each acknowledgement from the one or more communicators.

3. Distributed communication system according to claim 1 or claim 2, wherein the control device is configured to maintain a list of active communications, wherein a communication is entered onto the list upon receipt of the corresponding message with identificator and, optionally, is removed from the list upon receipt of the corresponding acknowledgement, and/or
wherein the receiver further is configured to check the integrity of each message from the one or more sources and of each acknowledgement of the one or more communicators, and preferably to raise an alarm condition when a checked message and/or acknowledgement is invalid.

4. Distributed communication system according to any one of the preceding claims, wherein each message from the one or more sources additionally comprises at least one of a sender ID and a timestamp, and/or
wherein the control device is configured to verify timely signaling of each communication from the one or more communicators, preferably by means of determining an expired amount of time between receipt of each of the identificators and receipt of the corresponding acknowledgement, and to raise an alarm condition when one or more communications from the one or more communicators are not timely signaled.

5. Distributed communication system according to any one of the preceding claims, wherein the control device is configured to verify correct signaling of each communication from the one or more communicators and/or correct communicating of each communication from the one or more sources, and to raise an alarm condition when one or more communications from the one or more communicators are not correctly signaled, and/or when one or more communications from the one or more sources are not correctly communicated.

6. Distributed communication system according to claim 4 or claim 5, wherein the control device comprises a verifier configured to verify timely and/or correct signaling of each communication from the one or more communicators and/or correct communicating of each communication from the one or more sources.

7. Distributed communication system according to any of the preceding claims, wherein the control device comprises a logger and a storage for logging and storing messages received from the one or more sources and acknowledgements received from the one or more communicators, and/or
wherein the distributed communication system further comprises an indicating device for indicating when an alarm condition has been raised, preferably wherein the distributed communication system is a distributed alarm system.

8. Distributed communication system according to any one of the preceding claims, further comprising one or more integrators, wherein each of the one of more integrators is in data communication with the one or more sources, with the one or more communicators, and/or with one or more other integrators, wherein each of the one or more integrators is configured to receive at least one communication originated by at least one of the one or more sources and/or by at least one of one or more other integrators, and to forward the at least one received communication to the one or more communicators, and/or to one or more other integrators, preferably wherein each of the one or more integrators is configured to send a message to the control device to confirm that the at least one received communication has been forwarded or has been received, wherein the message comprises the identificator of the respective communication.

9. Distributed communication system according to any one of the preceding claims, comprising one or more sensors configured to verify the functioning of the one or more communicators, preferably wherein the one or more sensors are configured to raise an alarm condition when a communication failure occurs by at least one of the one or more communicators, and/or
wherein at least one of the one or more communicators is configured to support operator confirmation, preferably wherein the at least one of the one or more communicators is configured to transmit the operator confirmation to the control device.

10. Distributed communication system according to any one of the preceding claims, wherein each of the one or more sources comprises a source ID, wherein each identificator included by the one or more sources comprises an associated source ID and a local ID, preferably wherein a value of the local ID is incremented or decremented each time a subsequent identificator is included in a message, preferably wherein the value of the local ID is sequential, and/or
wherein the one or more sources are configured for communicating a test communication, preferably periodically, and wherein the one or more communicators are configured to acknowledge receipt of such test communication, preferably automatically.

11. Method of controlling a distributed communication system, wherein the distributed communication system comprises:
- one or more sources configured to communicate one or more communications when triggered thereto;
- one or more communicators configured to receive one or more communications from the one or more sources, and to signal the received one or more communications to one or more recipients; and
- a control device configured to track communications originating from the one or more sources, wherein the method comprises the steps of:
- at each source including an identificator into each communication originating therefrom;
- for each communication, transmitting a message with the associated identificator to the control device;
- transmitting an acknowledgment to the control device regarding signaling of a received communication, including the identificator associated with the signaled communication;
- tracking communications within the distributed communication system by means of received associated identificators; and
- raising an alarm condition upon non-arrival of associated communications at any of the one or more communicators.

12. Method according to claim 11, wherein each of the one or more sources comprises a source ID, wherein the step of at each source including an identificator into each communication originating therefrom comprises the step of combining the source ID and a local ID into an identificator, preferably wherein the local ID has a value, wherein the method further comprises the step of incrementing or decrementing the value of the local ID for each subsequent identificator to be included, preferably wherein the local ID is sequential.

13. Method according to any of the claims 11-12, wherein the control device is configured to maintain a list of active communications, wherein the method comprises the steps of adding a communication to the list of active communications upon receipt of a message from the one or more sources and closing the communication upon receipt of an acknowledgement of the one or more communicators acknowledging signaling of the associated communication, and/or
wherein the distributed communication system comprises one or more integrators, wherein each of the one of more integrators is in data communication with the one or more sources, with the one or more communicators, and/or with one or more other integrators, wherein each of the one or more integrators is configured to receive at least one communication originated by at least one of the one or more sources and/or by at least one of one or more other integrators, and to forward the at least one received communication to the one or more communicators, and/or to one or more other integrators, preferably wherein each of the one or more integrators is configured to send a message to the control device to confirm that the at least one received communication has been forwarded or has been received, wherein the message comprises the identificator of the respective communication.

14. Method according to claim 13, comprising the step of updating the list of active communications upon receipt of a message of the one or more integrators, such that it is indicated in the list of active communications that the communication associated with the received message has reached or passed the integrator which has sent the message.

15. Computer-readable medium having computer-executable instructions adapted to cause a distributed communication system according to any one of the claims 1-10 to perform a method according to any one of the claims 11-14.
